# EUROPEAN PATENT SPECIFICATION

(11) **EP 2 526 931 B1**
(45) Date of publication and mention of the grant of the patent: **17.12.2014**
(21) Application number: 11167115.2
(22) Date of filing: 23.05.2011
(51) Int. Cl.: A61K 9/16, A61K 31/23

(54) **Wet granulation methods of cetyl myristate and/or cetyl palmitate**
Nasse Granulationsmethoden für Cetylmyristat und/oder Cetylpalmitat
Procédé pour la granulation humide de cetyl myristate et/ou cetyl palmitate

(43) Date of publication of application: 28.11.2012
(73) Proprietor: Deva Holding Anonim Sirketi, 34303 Istanbul (TR)
(72) Inventor: Haas, Philipp Daniel, 34303 Istanbul (TR); Firat, Omer Faruk, 34303 Istanbul (TR); Sivasligil, Ramazan, 34303 Istanbul (TR); Koc, Fikret, 34303 Istanbul (TR); Kandemir, Levent, 34303 Istanbul (TR)

(56) References cited:
- WO-A1-01/85162
- WO-A2-02/45686
- DE-A1- 3 523 454
- ANONYMOUS: "Cetyl Myristoleate Powder Specification Sheet", INTERNET CITATION, 31 July 2007 (2007-07-31), pages 1-2, XP002632858, Retrieved from the Internet: URL:http://www.essentiallypure.com/cetyl_m yristoleate_spec.htm [retrieved on 2011-04-14]
- Anonymous: "Methanol - Wikipedia", , 5 March 2011 (2011-03-05), XP002660867, Retrieved from the Internet: URL:http://web.archive.org/web/20110305133 347/http://en.wikipedia.org/wiki/Methanol [retrieved on 2011-10-10]

## Description

### Technical Field

This invention is related to optimization of granulation for manufacturing of cetyl myristate or cetyl palmitate or combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to reacting palmitoyl chloride and cetyl alcohol in the presence of magnesium. See the Merck Index, 12th edition at page 336. Reference is also made to US 3169099 A (SOCONY MOBIL OIL CO INC) 02.09.1965 patent which discloses a biosynthetic method of producing cetyl palmitate.

Cetyl myristate is derived from the saturated fatty acid, myristic acid. This acid is found in nutmeg butter, in fats of Myristicaceae, in palm seed fats, milk fats and also sperm whale oil.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt. % of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate. That mixture comprises by weight 95% cetyl myristate and 5% cetyl palmitate by weight. In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/ or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (e.g; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate. The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt. %) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention is related to optimization of granulation for manufacturing of cetyl myristate or cetyl palmitate or combination of cetyl myristate and cetyl palmitate.

### Technical Problem

In pharmaceutical manufacturing, wet granulation method is widely used due to consistent and high quality granular product. Wet granulation still remains to be an attractive technique to improve content uniformity, flow, compressibility properties of the powder blend, prevents segregation. This process involves several steps such as milling, dry mixing, wet massing, drying and milling of powder.

A variety of wet granulation process technique is available in the production of pharmaceutical oral dosage forms. Based on the principle of operations, three main techniques such as high shear granulation; low shear granulation and fluid bed granulation are commonly used.

The mechanical properties of the tablet may be under influence of either operating variables or formulation of the product. Especially for hardly processable active ingredients where formulation improvements are not conclusive, manufacturing difficulties may be eliminated by optimization of processing technique and variables. A well defined granulation process decreases variability in process and improve reproducibility of product.

Selection of the correct granulation technique and/or equipment is mainly based on active ingredient and desired product characteristics.

Moreover, changing granulation technique will have major effect on product properties, because different granulation systems show markedly different pattern of powder wetting- granule growth-granule consolidation-granule breakage steps which directly affect product quality. Final granule characteristics resulting from different techniques are effortful to compare because it is often difficult for the same formulation to be successfully processed through different granulation methods.

Nowadays, transfer of technology become important in pharmaceutical industry. And the implementation of successful transfer is required by minimization of site changes related problems. Changes or variations in the manufacturing process causes out of specifications and/or out of trend results. Similarly, although basic granulation technique may be the same, resulting granules may differ from site to site during technology transfer.

A consistent and uniform granulation process and granules of optimal quality can be achieved when the effects of critical parameters are controlled. Although process parameters including type and speed of impeller, surface area of impeller, proportionality between surface area of impeller and total volume in which granulation takes place, type and amount of binder, liquid addition rate, wet massing time, type and speed of chopper, timing and duration of chopper, mixing time after addition of binder which are all critical for product quality, granulating liquid and/or binding agent influence primarily resulting granules in all types of granulation.

In the case of granulation of waxy compound such as cetyl myristate and cetyl palmitate, granulating agent has a critical importance and must be chosen carefully. In such formulation, water and mixtures are avoided as granulating agent; preparation of pharmaceutical formulation becomes nearly impossible due to their gelling feature.

In all wet granulation techniques, the amount of solvent has also strong influence on the tablet properties.

Subsequently, optimization of granulation process is necessary to improve quality of granules and finished products for all wet granulation techniques for compositions containing cetyl myristate and cetyl palmitate.

### Solution to Problem

Optimization of granulation process of oral solid dosage forms of cetyl myristate and/or cetyl palmitate or combination of cetyl myristate and cetyl palmitate is needed on a commercial scale, and achievement of same product quality through different granulation techniques is aimed by this invention.

A simple and reproducible granulating process for oral solid dosage forms of cetyl myristate and/or cetyl palmitate or combination of cetyl myristate and cetyl palmitate is required to be developed to overcome problems that are encountered in manufacturing with all wet granulation techniques.

Inventors of the present invention investigated the influence of granulation parameters on manufacturing of cetyl myristate and cetyl palmitate granules and optimized granulation processor robust, reproducible and scalable manufacturing process.

A granulation technique independent process is developed by inventors for oral solid dosage forms of cetyl myristate and/or cetyl palmitate or combination of cetyl myristate and cetyl palmitate to minimize production site and granulation related variations.

Moreover, it is known that good selection and amount of granulation solvent is especially critical for a granulation process of waxy materials. Therefore, granulation solvent and parameters should be selected according to API's characteristics as well.

Inventors of this invention found that encountered difficulties in the granulation of cetyl myristate and/or cetyl palmitate or combination of cetyl myristate and cetyl palmitate can be surpassed by two different methods.

Firstly, use of isopropyl alcohol as granulation solvent is surprisingly found to exhibit improved features when compared with other granulation solvents in all granulation techniques. Secondly, use of organic volatile solvent is found to enable controlled fluid bed granulation through modifications in process design.

The invention therefore relates in a first aspect to a wet granulation method of cetyl myristate or cetyl palmitate or combination of cetyl myristate and cetyl palmitate, characterized in that granulation solvent is isopropyl alcohol wherein granulation is performed in low shear and/or high shear and/or fluid bed granulation. In a second aspect, the invention relates to a wet granulation method of cetyl myristate or cetyl palmitate or combination of cetyl myristate and cetyl palmitate, characterized in that a volatile organic solvent selected from the group consisting of dichloromethane, methanol, acetone, or mixtures thereof is used as a granulating agent and fluid bed drying is performed while the inlet air of fluid bed granulator is cooled to 20°C with an internal chiller or chilled water in course of drying of granules.

It is also found that granules prepared with isopropyl alcohol in all granulation techniques and dichloromethane in fluid bed granulation show better properties at processibility, reproducibility, removal of residual solvent and powder flow features for oral solid dosage forms of cetyl myristate or cetyl palmitate or combination of cetyl myristate and cetyl palmitate.

### Description of embodiments

In the first aspect of this invention, the present inventors have performed aqueous and organic solvent wet granulations for cetyl myristate and cetyl palmitate and cetyl myristate and cetyl palmitate combination. The terms of "cetyl myristate" and "cetyl palmitate" and "combination of cetyl myristate and cetyl palmitate" cover all of the pharmaceutical or dietary purposes.

Final granules resulting from high shear, low shear and fluid bed granulations of cetyl myristate and cetyl palmitate combinations are evaluated and critical parameters have been determined for the process.

Consequently, the influence of drying time of granules, residual solvent in granules and angle of repose of granules on finished products are investigated for all wet granulation methods by using different solvents.

Drying time was identified as high risk factor for residual solvent, especially considering the fact that drying is done at a much lower temperature due to the low melting point of the API. The drying process can impact the properties, functionality and quality of the material being dried. Therefore, it is necessary to optimize the drying time to ensure the quality of the product.

Granulation is done at ambient temperature, and the drying temperature has been optimized at 35°C.

Residual solvent presence in a final product is undesirable and should be avoidable. Safe and effective methods have to be used to remove them. It is impossible to completely remove all traces of the organic solvent or alcohol from the granulated drug substance. Trace solvents can leave adverse odours in the finished dosage form that are objectionable to many patients.

The solvent used in granulation process is generally a volatile hydrocarbon or alcohol which can easily be removed from the granules after they are formed. However, the use of highly volatile solvents presents a new set of problems. These solvents are generally environmentally deleterious and have a great risk of explosion during handling them.

Regulation has a strict limitation of the solvent residue in some granule products, such as in pharmaceuticals and food products. Published pharmacopeias and ICH guidelines determine maximum allowable amounts of residual solvent in pharmaceutical products.

The angle of repose is defined as the angle of free surface of a pile of powder to the horizontal plane. In the present invention, angle of repose is determined according to US Pharmacopeia General Chapter<1174>.

In the present study, angles of repose are measured for all of these formulations and flow properties of granules resulting from different granulations are judged from angles of repose.

The results shown in table 1 reveal that granules prepared by different granulating solvents and granulation techniques show different features.

**Table 1**

| | Drying Time* | | | Residual Solvent | | | Angle of Repose | | |
|---|---|---|---|---|---|---|---|---|---|
| | LSG | HSG | FBG | LSG | HSG | FBG | LSG | HSG | FBG |
| water | 126 | 148 | 231 | Less than 1.0% (w/w) | | | 45 | 35 | 39 |
| ethanol | 73 | 84 | 164 | Less than 0.5% (w/w) | | | 50 | 44 | 42 |
| dichlorom ethane | 29 | 35 | ** | Less than 0.1 % (w/w) | | | *** | 57 | ** |
| IPA | 87 | 96 | 179 | Less than 0.3% (w/w) | | | 33 | 21 | 24 |
| methanol | 41 | 52 | 85 | Less than 0.1 % (w/w) | | | 54 | 49 | 45 |
| acetone | 40 | 49 | 77 | Less than 0.1 % (w/w) | | | 51 | 36 | 42 |

* Drying time means total time spent for drying wet granules of low shear or high shear granulators. For fluid bed granulator, drying time is equal to total processing time.

**Granulation cannot be completed at ambient temperature.

*** Angle of repose cannot be measured with standard orifice that is used for solvents.

For selection of best granulation solvent, the results are compared as follows.

**Water as granulating agent:** The present inventors have found out that although granules prepared by high shear granulation and fluid bed granulation show good flowing properties, low shear granulation's granules have a large angle of repose which has been judged as a sign of poor flowability. Besides, fluid bed granulation is not suitable by reason of long drying time. It is realized that active ingredients are sticking on the granulator internal walls throughout the process. Consequently, water is not preferable for all granulation techniques.

**Ethanol as granulating agent:** It is found that low shear, high shear and fluid bed granulation techniques are not suitable due to the fact that corresponding angles of repose are exceeding 40° which does not satisfy flow properties of granules of cetyl myristate and cetyl palmitate and cetyl myristate and cetyl palmitate combination. Consequently, low flowability of granules hampers powder feeding in tablet compression and in capsulation.

**Isopropyl alcohol as granulating agent:** The inventors of the present invention have surprisingly found that using of isopropyl alcohol as granulating agent shows better flowability properties in all granulation techniques when compared with other granulation solvents.

Besides, use of IPA reduces drying time for making granules which provides a shorter and more reproducible process. Residual solvent analysis indicated the use of IPA which is easier to remove as compared to water.

**Dichloromethane, Methanol, Acetone as granulating agent:** The fluid bed granulation with dichloromethane cannot be completed due to the low boiling point of granulating solvent.

The inventors have scoped out the occurring problem and figured out by adjustments in granulation equipment and process. In spraying, it is observed that dichloromethane is immediately lost by evaporation.

In this respect, wetting of powder by dichloromethane, methanol and acetone does not occur and powder to be granulated is only aerated without granulation. It is found that incoming air of fluid bed granulation process must be adjusted. Considering the product being granulated, the inlet air temperature should be set to 20°C in fluid bed drying of cetyl myristate and cetyl palmitate or combination of cetyl myristate and cetyl palmitate.

It is proposed that the inlet air temperature of fluid bed granulator used for drying is cooled with an internal chiller or chilled water.

From above mentioned granulating solvents, isopropyl alcohol and volatile organic solvent such as dichloromethane, methanol, acetone or mixtures thereof are chosen for further investigation and optimization of the process.

Total amount of dichloromethane, methanol, acetone or mixtures thereof is from about 20% to about 40%, more preferably from %25 to about %35 of total mass of powder mixture to be granulated.

In another aspect of this invention, critical parameters such as impeller speed, solvent quantity and solvent addition rate variations are investigated on finished product properties.

IPA quantity should be set in order to minimize by reasons of safety and quality purposes. Inventors defined a specific range for using isopropyl alcohol.

Liquid dosing rate controls granule density and using excessive levels of granulating fluids and binding agent may give rise to sticking to internal wall of granulator which ends up in a low yielded production. Nevermore, formation of lump and non-homogeneous granules should not be disregarded.

Other process parameters such as over mixing of excipients or slow evaporation of any excess granulating fluid similarly cause formation of hard granules. Tablets comprising such hard granules may be also undesirably hard which are very difficult to disintegrate or capping during compression occurs.

A randomized Fractional Factorial Design (2⁵⁻¹) with 3 center points (16+3=19 batches) was generated using Design Expert (Ver.8) software. The results of the 19 batches have been summarized as follows:

**Table 2**

| | **Impeller Speed (rpm)** | **IPA Quantity (%)** | **IPA Addition rate (min)** | **Drying time (min)** |
|---|---|---|---|---|
| | 125 | 17.5 | 5 | 90 |
| | 100 | 27 | 2 | 125 |
| | 150 | 20 | 2 | 75 |
| | 100 | 15 | 8 | 75 |
| | 100 | 20 | 8 | 105 |
| | 150 | 20 | 8 | 105 |
| | 150 | 15 | 2 | 75 |
| | 150 | 20 | 8 | 75 |
| | 150 | 15 | 2 | 105 |
| | 100 | 15 | 8 | 105 |
| | 100 | 15 | 2 | 105 |
| | 100 | 20 | 2 | 75 |
| | 150 | 15 | 8 | 105 |
| | 150 | 15 | 8 | 75 |
| | 100 | 15 | 2 | 75 |
| | 150 | 20 | 2 | 105 |
| | 100 | 20 | 8 | 75 |
| | 125 | 17.5 | 5 | 90 |
| | 125 | 17.5 | 5 | 90 |

The results shown in table 2 reveals that the total amount of isopropyl alcohol that should be used as granulating agent, is from about 10% to about 25%, more preferably from %15 to about %20 of total mass of powder mixture to be granulated.

In another aspect of the present invention, the compositions of cetyl myristate and cetyl palmitate may include fillers, binders, disintegrants, glidants, lubricants, flavourings.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, α-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested .Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Flavourings are, but not limited to, cinnamon oil, essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehydeacacia, anise oil, benzaldehyde, caraway oil, cardamom (oil, tincture, spirit),cherry syrup, citric acid syrup, citric acid, clove oil, cocoa, cocoa syrup, coriander oil, dextrose, ethyl acetate, ethyl vanillin, fennel oil, ginger, glucose, glycerin, glycerrhiza, honey, lavender oil, lemon oil, mannitol, nutmeg oil, methyl salicylate, orange oil, orange flower water, peppermint (oil, spirit, water), raspberry, rose (oil, water), rosemary oil, saccharin sodium, Sarsaparilla syrup, spearmint oil, thyme oil, tolu balsam, vanilla ,vanilla tincture, tolu balsam syrup, wild cherry syrup and mixtures thereof and the like.

Suitable pharmaceutical compositions include, but are not limited to, capsules, tablets, granules, powders and unit dose pockets.

The following example is given for the purpose of illustration of the present invention and should not limit the scope of the invention.

### Example: Capsule Unit Formula and Manufacturing Process:

There is provided a process for the capsule preparation of a pharmaceutical form of cetyl myristate or cetyl palmitate or combination of cetyl myristate and cetyl palmitate, suitable for oral administration, said process comprising following steps:

a) 700.00 mg per capsule of active ingredients and 127.2 mg per capsule of diluent, 17.63 mg per capsule of disintegrant, 47.00 mg per capsule of binder are weighed and sifted using a suitable screen in Vibratory sifter.

b) Sifted diluents, active ingredients, disintegrant and binder are transferred to Granulator and premixed.

c) Granulation is performed with a granulation agent and granules are dried in Vector VFC Flo-Coater 30x fluid bed granulator.

d) Mill the granules retained on Vibratory sifter through suitable screen in a Quadro co-mill to break small lumps / larger granules.

e) Granules are stored in air tight polyethylene container lined with double poly bag and protected from light.

f) Fill and collect capsules after checking the following: Individual weight variation, group weight and disintegration time.

## Claims

1. A wet granulation method of cetyl myristate or cetyl palmitate or combination of cetyl myristate and cetyl palmitate **characterized in that** granulation solvent is isopropyl alcohol wherein granulation is performed in low shear and/or high shear and/or fluid bed granulation.

2. The wet granulation method according to claim 1, wherein isopropyl alcohol is used from 10% to 25%, more preferably from 15% to 20% of total mass of powder mixture to be granulated.

3. A wet granulation method of cetyl myristate or cetyl palmitate or combination of cetyl myristate and cetyl palmitate **characterized in that** volatile organic solvent selected from the group consisting of dichloromethane, methanol, acetone or mixtures thereof are used as granulating agent and fluid bed drying is performed while the inlet air of fluid bed granulator is cooled to 20°C with an internal chiller or chilled water in course of drying of granules.

4. The wet granulation method according to claim 3, wherein dichloromethane, methanol, acetone or mixtures thereof is used from 20% to about 40%, more preferably from 25% to 35% of total mass of powder mixture to be granulated.

## Patentansprüche

1. Ein Nassgranulationsverfahren von Cetylmyristat oder Cetylpalmitat oder Kombination von Cetylmyristat und Cetylpalmitat, **gekennzeichnet dadurch, daß** es sich bei dem Granulatlösungsmittel um Isopropylalkohol handelt, wobei die Granulation als niedrige Scher-und/oder höhere Scher- und/oder Wirbelbettgranulation durchgeführt wird.

2. Nassgranulationsverfahren nach Anspruch 1, wobei Isoprpylalkohol in einem Anteil von 10% bis 25 %, besonders bevorzugt von 15% bis 20% der Gesamtmasse des zu granulierenden Pulvergemisches beträgt.

3. Ein Nassgranulationsverfahren von Cetylmyristat oder Cetylpalmitat oder Kombination von Cetylmyristat und Cetylpalmitat, **gekennzeichnet dadurch, daß** als Granuliermittel flüchtiges organisches Lösemittel, ausgewählt aus der Gruppe bestehend aus Dichlormethan, Methanol, Aceton oder Gemischen davon eingesetzt werden, und Wirbelbetttrocknung durchgeführt ist, während die Ansaugluft in dem Wirbelbettgranulator, im Verlauf der Trocknung von Granulaten, mittels eines internen Kühlers oder gekühlten Wassers auf 20°C gekühlt wird.

4. Nassgranulationsverfahren nach Anspruch 3, wobei Dichlormethan, Methanol, Aceton oder Gemischen davon in einem Anteil von 20% bis etwa 40%, besonders bevorzugt von 25% bis 35% der Gesamtmasse des zu granulierenden Pulvergemisches eingesetzt wird.

## Revendications

1. Une méthode de granulation humide de myristate de cétyle ou palmitate de cétyle ou une combinaison de myristate de cétyle et de palmitate de cétyle **caractérisés en ce que** le solvant de granulation est d'alcool isopropylique dans lequel la granulation est effectuée en faible cisaillement et/ou haut cisaillement et/ou granulation à lit fluidisé.

2. La méthode de granulation humide suivant la revendication 1, où l'alcool isopropylique est utilisé de 10% to 25%, de préférence de 15% à 20% de la masse totale du mélange pulvérulent à agréger.

3. Une méthode de granulation humide de myristate de cétyle ou palmitate de cétyle ou une combinaison de myristate de cétyle et de palmitate de cétyle **caractérisés en ce que** le solvant organique volatil sélectionné dans le groupe se composant de dichlorométhane, de méthanol, d'acétone ou de leur mélange sont utilisés comme agent de granulation et où le séchage du lit fluidisé est effectué tandis que l'air d'entrée du granulateur de lit fluidisé est refroidi à 20°C avec un refroidisseur interne ou de l'eau réfrigérée au cours du séchage des granulats.

4. La méthode granulation humide suivant la revendication 3, où du dichlorométhane, du méthanol, de l'acétone ou leur mélange est utilisé de 20% à environ 40%, de préférence de 25% à 35%, de la masse totale du mélange pulvérulent à granuler.
